(19) Europäisches Patentamt
European Patent Office
Office européen des brevets

(11) EP 2 465 849 A1

(12) # EUROPEAN PATENT APPLICATION

(43) Date of publication:
**20.06.2012 Bulletin 2012/25**

(51) Int Cl.:
***C07D 213/78*** *(2006.01)* ***B01D 53/14*** *(2006.01)*

(21) Application number: **10015426.9**

(22) Date of filing: **08.12.2010**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Lonza Ltd**
**4052 Basel (CH)**

(72) Inventor: **The designation of the inventor has not yet been filed**

(54) **3-cyanopyridinium tricyanomethanides as ionic liquids**

(57) Disclosed are 3-cyanopyridinium tricyanomethanides of formula

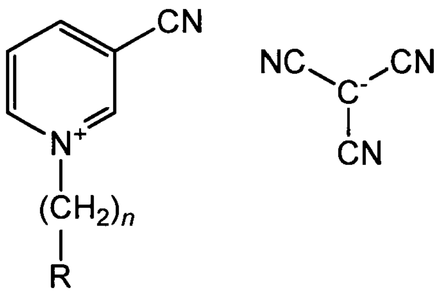

(I),

wherein *n* is an integer from 2 to 10 and R is hydrogen or -CN, and a process for the preparation thereof. The 3-cyanopyridinium tricyanomethanides are useful e. g. as selective solvents in the separation of alkene-alkane mixtures.

EP 2 465 849 A1

## Description

### Field of the Invention

The invention relates to 1-alkylpyridinium tricyanomethanides having a cyano group in position 3 of the pyridinium moiety. These compounds can be represented by the formula

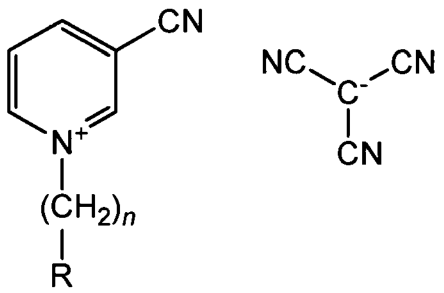

(I),

wherein *n* is an integer from 2 to 10 and R is either hydrogen or a cyano group. It further relates to a process for the production of said tricyanomethanides, 1-alkylpyridinium bromides as intermediates in said process, and the use of the tricyanomethanides as selective solvents in the separation of alkene-alkane mixtures.

### Background of the Invention

Tricyanomethanides (tricyanomethides) of organic cations such as 1,3-dialkylimidazolium are low-melting salts which are composed of carbon, hydrogen and nitrogen only. Some of them are already known as "ionic liquids". Ionic liquids (IL) are defined as materials that are composed only of ions and have a melting point below 100 °C. Besides a low vapor pressure, ionic liquids have unique physical and chemical properties that can be tuned by the selection of the cations and anions. Therefore, ionic liquids have also been termed "designer solvents". The term task specific ionic liquids (TSIL) has been coined for ionic liquids containing rather complex ions with additional functional groups that provide for specific properties.

The unique properties of ionic liquids allow for a plethora of potential applications (see e.g. N. V. Plechkova, K. R. Seddon, Chem. Soc. Rev. 2008, 37, 123-150). In fact, ionic liquids have been applied in synthesis, catalysis, analytics, biotechnology, process engineering, material science, and electrochemistry.

WO 2006/021390 A1 discloses several tricyanomethanides of 1,3-dialkylimidazolium and 1-alkylpyridinium cations. It had further been found that ionic liquids with tricyanomethanide as anion have interesting properties which allow to use them as selective solvents for the separation of mixtures which are hardly separable by simple distillation, such as propene-propane mixtures (V. Mokrushin et al., Chem. Eng. Technol., 2010, 33, 63-73).

It was an object of the present invention to provide new organic tricyanomethanides having functional groups which might be able to interact with compounds such as alkenes, thus improving the separation factor in alkene-alkane separations. It was a further object of the invention to provide a simple process for the preparation of said tricyanomethanides from easily available starting materials.

### Summary of the Invention

According to the invention, the above object has been achieved by the 3-cyanopyridinium tricyanomethanides of formula

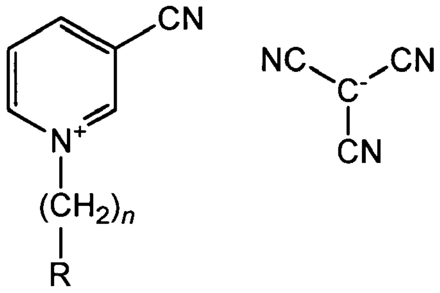

(I),

wherein *n* is an integer from 2 to 10 and R is either hydrogen or a cyano group.

The 3-cyanopyridinium tricyanomethanides of formula I can be prepared by a process comprising the steps of

(i) quaternizing 3-cyanopyridine by reaction with a bromide of formula

$$Br\text{-}(CH_2)_n\text{-}R\,, \qquad (II)$$

wherein *n* and R are as defined above, to obtain a 3-cyanopyridinium bromide of formula

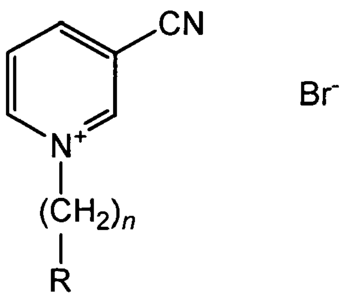

(III),

wherein *n* and R are as defined above, and

(ii) reacting the 3-cyanopyridinium bromide (III) with an alkali tricyanomethanide to obtain the 3-cyanopyridinium tricyanomethanide (I) and an alkali bromide, and

(iii) isolating the 3-cyanopyridinium tricyanomethanide (I).

The 3-cyanopyridinium bromides of formula III are also an object of the present invention.

<u>Detailed Description of the Invention</u>

In a preferred embodiment, *n* in formula I is an integer from 2 to 4.

Particularly preferred tricyanomethanides of formula I are 1-butyl-3-cyanopyridinium tricyanomethanide (*n*= 4, R = H), 1-(2-cyanoethyl)-3-cyanopyridinium tricyanomethanide (*n* = 2, R = -CN) and 1-(3-cyanopropyl)-3-cyanopyridinium tricyanomethanide (*n* = 3, R = -CN).

The quaternization step (i) in the process for preparing the tricyanomethanides of the invention is advantageously carried out in a polar solvent. Suitable polar solvents are for example esters, such as ethyl acetate or butyl acetate, ethers such as tetrahydrofuran or dioxane, or nitriles such as acetonitrile, propionitrile or butyronitrile. Acetonitrile is particularly preferred.

The reaction temperature is preferably in the range of 60-150 °C. If the reaction temperature is higher than the boiling point of the solvent at atmospheric pressure, the quaternization step is advantageously carried out in a sealed vessel such as an autoclave.

3-Cyanopyridine and the bromides of formula II are known compounds which are commercially available or can be prepared according to methods known in the art.

The ion exchange step (ii) can be carried out with the tricyanomethanide of any alkali metal, namely lithium, sodium, potassium, rubidium or cesium tricyanomethanide. The preferred tricyanomethanide is sodium tricyanomethanide which can be prepared according to known methods (see e.g. Schmidtmann, Chem. Ber. 1896, 29, 1168-1175).

The ion exchange step (ii) is advantageously carried out in a polar solvent wherein both the 3-cyanopyridinium bromide of formula III and the alkali tricyanomethanide are soluble. The preferred solvent is water.

The isolation of the tricyanomethanide of formula I in step (iii) can be achieved by methods known in the art, for example by extracting the aqueous reaction mixture with a solvent that is not water-miscible and wherein the tricyanomethanide is soluble but the alkali bromide formed as byproduct has negligible solubility, or by concentrating the reaction mixture and triturating the residue with a solvent that dissolves the tricyanomethanide but not the alkali bromide. A solvent that is suitable for both methods is ethyl acetate.

In analogy to the particularly preferred 3-cyanopyridinium tricyanomethanides, the most preferred 3-cyanopyridinium bromides of formula III are 1-butyl-3-cyanopyridinium bromide ($n$= 4, R = H), 1-(2-cyanoethyl)-3-cyanopyridinium bromide ($n$= 2, R = -CN) and 1-(3-cyanopropyl)-3-cyanopyridine ($n$ = 3, R = -CN).

A preferred use of the 3-cyanopyridinium tricyanomethanides of the invention is as a selective solvent in the separation of alkene-alkane mixtures.

In a preferred embodiment of said use, the 3-cyanopyridinium tricyanomethanide of formula I is the compound wherein $n$ is 4 and R is hydrogen, namely 1-butyl-3-cyanopyridinium tricyanomethanide.

A preferred alkene-alkane mixture which can be separated according to the present invention is a mixture of propene and propane.

The following examples will illustrate the invention in more detail, however they are not intended to limit the present invention.

### Example 1

**1-Butyl-3-cyanopyridinium bromide (III, *n* = 4, R = H)**

A solution of 3-cyanopyridine (249.9 g, 2.38 mol, 1 eq) in acetonitrile (700 g) was charged to a 3 L stainless steel vessel equipped with pressure sensor and thermometer. After the air in the vessel had been replaced with nitrogen, the solution was heated to 120 °C whereby the internal pressure reached about 3.8 bar. Then 1-bromobutane (400 g, 2.9 mol, 1.2 eq) was pumped into the vessel at 120 °C within 2 h and the reaction temperature was maintained at 120 °C for another 16 h. Then the mixture was cooled from 120 °C to 25 °C within 3 h, whereby most of the pyridinium bromide crystallized quickly at about 76 °C. The product was filtered off and washed 3 times with acetone (800 g) to remove the acetonitrile. The solid was dried overnight at 80 °C under vacuum (10 mbar).

Yield: 473 g (82.6%) white powder. m.p.: 148-149 °C.
$^1$H NMR (400 MHz, DMSO-$d_6$): δ 9.92 (s, 1H), 9.46 (d, $J$= 6.0 Hz, 1H), 9.12 (d, $J$= 6.0 Hz, 1H), 8.37 (dd, $J$= 6.0, 7.6 Hz, 1H), 4.68 (t, $J$= 7.6 Hz, 2H), 1.93 (m, 2H), 1.32 (m, 2H), 0.93 (t, $J$= 7.2 Hz, 3H).
$^{13}$C NMR (100 MHz, DMSO-$d_6$): δ 149.35, 148.78, 148.62, 128.83, 114.26, 112.94, 61.40, 32.66, 19.05, 13.75.

### Example 2

**1-Butyl-3-cyanopyridinium tricyanomethanide** (I, $n$ = 4, R = H)

1-Butyl-3-cyanopyridinium bromide (20 g, 82 mmol, 1 eq) was reacted with sodium tricyanomethanide (10.2 g, 86 mmol, 1.05 eq) in water (50 g) at 50 °C for 4 h. Two layers formed during the reaction. Ethyl acetate (61 g) was added to the reaction mixture and the organic phase was separated and washed three times with water (30 g). The ethyl acetate was finally evaporated under vacuum (10 mbar) at 80 °C to obtain the tricyanomethanide as a clear brown liquid. Yield: 16.1 g (78%).

### Example 3

**1-Butyl-3-cyanopyridinium tricyanomethanide** (I, $n$ = 4, R = H)

A solution of 1-butyl-3-cyanopyridinium bromide (500 g) in water (200 mL) was treated with a solution of sodium tricyanomethanide (235.1 g, 1 eq.) in water (800 mL). The resulting 1-butyl-3-cyanopyridinium tricyanomethanide formed a second liquid layer. The reaction mixture was extracted with ethyl acetate (3x300 mL). The combined organic phase was washed with water (5x200 mL), and the solvent was evaporated. The residue was dried *in vacuo* to give 1-butyl-3-cyanopyridinium tricyanomethanide. Yield: 474.2 g (91 %). m.p. (DSC): 45 °C.
Water content (Karl Fischer titration): 967 ppm.
$^1$H NMR (400 MHz, DMSO-$d_6$): δ 9.801 (s, 1H), 9.330 (d, $J$= 6.2 Hz, 1H), 9.071 (d, $J$= 8.2 Hz, 1H), 8.355 (dd, $J$= 6.2, 6.2 Hz, 1H), 4.621 (t, $J$= 7.4 Hz, 2H), 1.936 (quintet, $J$= 7.4 Hz, 2H), 1.313 (sextet, $J$= 7.4 Hz, 2H) 0.925 (t, $J$= 7.4 Hz, 3H).
$^{13}$C NMR (100 MHz, DMSO-$d_6$): δ 149.36, 148.74, 148.64, 128.96, 120.95, 114.17, 113.60, 62.13, 32.77, 19.21, 13.68,

5.35.

**Example 4**

**1-(2-Cyanoethyl)-3-cyanopyridinium bromide** (III, *n* = 2, R = CN)

3-Cyanopyridine (30 g, 288 mmol) and 3-bromopropionitrile (42.466 g, 317 mmol) were dissolved in acetonitrile (150 mL). The solution was sealed in an autoclave, heated to 75 °C, and kept stirring at this temperature for 100 h. Then the reaction mixture was cooled down to room temperature, the precipitated solid was filtered off, washed with acetonitrile, and dried *in vacuo* for 3 h at 80 °C to give a white solid.
Yield: 22 g (32%).
$^1H$ NMR (400 MHz, DMSO-$d_6$): δ 9.976 (s, 1H), 9.479 (d, *J*= 6.4 Hz, 1H), 9.193 (m, 1H), 8.472 (m, 1H), 5.037 (t, *J*= 6.8 Hz, 2H), 3.464 (t, *J*= 6.8 Hz, 2H).

**Example 5**

**1-(3-Cyanopropyl)-3-cyanopyridinium bromide** (III, *n* = 3, R = CN)

3-Cyanopyridine (21.2 g, 204 mmol) and 4-bromobutyronitrile (31.7 g, 214 mmol) were dissolved in acetonitrile (1000 mL). The solution was sealed in an autoclave, heated to 120 °C, and kept stirring at this temperature for 144 h. Then the reaction mixture was cooled down to room temperature and the solvent was distilled off. The solid residue was dissolved in methanol and the product was precipitated by addition of diethyl ether. solid was filtered off and recrystallized from methanol and diethyl ether. The product was isolated by filtration, washed with diethyl ether, and dried at room temperature under a nitrogen flow for 3 h to afford a white solid.
Yield: 27 g (52.6%).
$^1H$ NMR (400 MHz, DMSO-$d_6$): δ 9.869 (s, 1H), 9.392 (d, *J*= 6.0 Hz, 1H), 9.137 (m, 1H), 8.398 (m, 1H), 4.735 (t, *J*= 7.2 Hz, 2H), 2.680 (t, *J*= 7.2 Hz, 2H), 2.341 (quintet, *J*= 7.2 Hz, 2H).

**Example 6**

**1-(3-Cyanopropyl)-3-cyanopyridinium tricyanomethanide** (I, *n* = 3, R = CN)

1-(3-Cyanopropyl)-3-cyanopyridinium bromide (1 g, 4 mmol) and sodium tricyanomethanide (0.561 g, 4.8 mmol) were dissolved in water (20 mL). The solution was heated to 50 °C, kept stirring at this temperature for 15 h, and then cooled down to room temperature. The water was distilled off as far as possible and the residue was triturated with ethyl acetate. The resulting suspension was filtered and the filtrate concentrated to obtain a light brown solid which was dried *in vacuo*.
Yield: 450 mg (~30%, containing ~20% NaTCM).
m.p. >150°C.
$^1H$ NMR (400 MHz, DMSO-$d_6$): δ 9.811 (s, 1H), 9.328 (m, 1H), 9.123 (m, 1H), 8.383 (m, 1H), 4.696 (t, *J*= 7.2 Hz, 2H), 2.654 (t, *J*= 7.2 Hz, 2H), 2.338 (quintet, *J*= 7.2 Hz, 2H).
$^{13}C$ NMR (100 MHz, DMSO-$d_6$): δ 149.92, 149.09, 129.00, 120.96, 119.99, 114.37, 113.35, 60.81, 26.21, 13.91, 5.22.

**Example 7**

**Determination of the ene/ane separation factor in the separation of propene and propane**

The separation factor $T_{i/j}$, for the separation of propene/propane was determined for 1-butyl-3-cyanopyridinium tricyanomethanide and several comparison compounds. The separation factor is defined as

$$T_{i/j} = (y_i \times x_j)/(x_i \times y_j),$$

where $y_i$ and $y_j$ designate the mole fractions of components i and j in the gas phase and $x_i$ and $x_j$ designate the mole fractions in the liquid phase.

The index i is conventionally assigned to the component having the higher vapor pressure (i.e., propene). In the absence of an ionic liquid the molar fraction of propene in the gas phase is higher than in the liquid phase, which

results in a separation factor >1. In the presence of an ionic liquid the molar fraction of propene in the gas phase is reduced to such an extent that it becomes lower than that of propane, resulting in a separation factor <1. The lower the separation factor, the better is the separation obtained.

Method:

In a GC vial, 5 mL of the ionic liquid were incubated at room temperature with 30 mL of a gaseous mixture of propene/propane. After equilibrium had been attained, the gas phase was analysed by GC to determine $y_i$ and $y_j$, while the values of $x_i$ and $x_j$ were calculated.

The following Table 1 shows the results for the separation of propene/propane by 1-butyl-3-cyanopyridinium tricyanomethanide (No. 1) and a range of other ionic liquids (Nos. C1 to C10), including both other tricyanomethanide ionic liquids (Nos. C7 to C9) and ionic liquids containing other anions (Nos. C1 to C6 and C10). Desirable is a low separation factor (<<1). Surprisingly, 1-butyl-3-cyanopyridinium tricyanomethanide ([Bu3-CNPy][$C(CN)_3$]) exhibits an unprecedentedly low separation factor.

The abbreviations used in Table 1 are as follows:

Cations:

[Bu3-CNPy] = 1-butyl-3-cyanopyridinium
[MMIM] = 1,3-dimethylimidazolium
[EMIM] = 1-ethyl-3-methylimidazolium
[BMIM] = 1-butyl-3-methylimidazolium
[BBIM] = 1,3-dibutylimidazolium
[EMMIM] = 1-ethyl-2,3-dimethylimidazolium
[Bu3-Pic] = 1-butyl-3-methylpyridinium

Anions:

[$C(CN)_3$] = tricyanomethanide
[$Tf_2N$] = bis(trifluoromethylsulfonyl)imide
[$Bu_2PO_4$] = dibutylphosphate
[$B(CN)_4$] = tetracyanoborate

Table 1

| No. | Name | wt.% (ene+ane) in liquid phase | ene/ane separation factor $T_{i/j}$ |
|---|---|---|---|
| 1 | [Bu3-CNPy][$C(CN)_3$] | 0.048 | **0.211** |
| C1 | [MMIM][$Tf_2N$] | 0.244 | 0.476 |
| C2 | [EMIM][$Tf_2N$] | 0.266 | 0.523 |
| C3 | [BMIM][$Tf_2N$] | 0.352 | 0.574 |
| C4 | [BBIM][$Tf_2N$] | 0.403 | 0.666 |
| C5 | [EBIM][[$Bu_2PO_4$] | 0.483 | 0.695 |
| C6 | [BBIM][[$Bu_2PO_4$] | 0.549 | 0.727 |
| C7 | [EMIM][$C(CN)_3$] | 0.367 | 0.466 |
| C8 | [EMMIM][$C(CN)_3$] | 0.305 | 0.445 |
| C9 | [Bu3-Pic][$C(CN)_3$] | 0.446 | 0.521 |
| C10 | [EMIM][$B(CN)_4$] | 0.500 | 0.407 |

## Claims

**1.** A 3-cyanopyridinium tricyanomethanide of formula

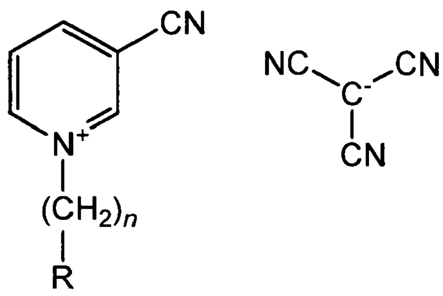

(I),

wherein *n* is an integer from 2 to 10 and R is hydrogen or -CN.

**2.** The 3-cyanopyridinium tricyanomethanide of claim 1, wherein *n* is an integer from 2 to 4.

**3.** 1-Butyl-3-cyanopyridinium tricyanomethanide.

**4.** 1-(2-Cyanoethyl)-3-cyanopyridinium tricyanomethanide.

**5.** 1-(3-Cyanopropyl)-3-cyanopyridinium tricyanomethanide.

**6.** A process for the preparation of a 3-cyanopyridinium tricyanomethanide of formula

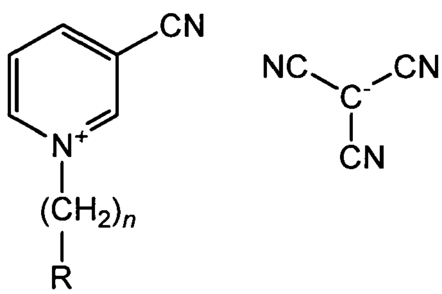

(I),

wherein *n* is an integer from 2 to 10 and R is hydrogen or -CN, comprising the steps of

(i) quaternizing 3-cyanopyridine by reaction with a bromide of formula

$Br-(CH_2)_n-R$ , (II)

wherein *n* and R are as defined above, to obtain a 3-cyanopyridinium bromide of formula

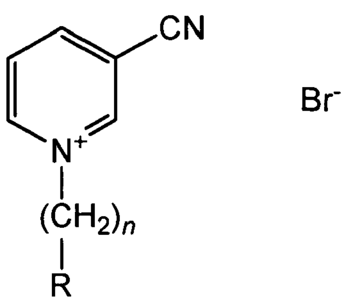

(III),

wherein *n* and R are as defined above, and

(ii) reacting the 3-cyanopyridinium bromide (III) with an alkali tricyanomethanide to obtain the 3-cyanopyridinium tricyanomethanide (I) and an alkali bromide, and
(iii) isolating the 3-cyanopyridinium tricyanomethanide (I).

**7.** The process of claim 6, wherein the quaternization step (i) is conducted in acetonitrile.

**8.** The process of claim 6 or 7, wherein the alkali tricyanomethanide in step (ii) is sodium tricyanomethanide.

**9.** The process of any of claims 6 to 8, wherein the reaction with the alkali tricyanomethanide in step (ii) is conducted in water.

**10.** A 3-cyanopyridinium bromide of formula

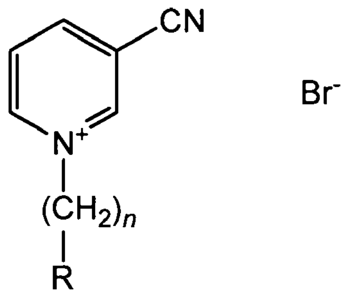

(III),

wherein *n* is an integer from 2 to 10 and R is hydrogen or -CN.

**11.** Use of a 3-cyanopyridinium tricyanomethanide of formula

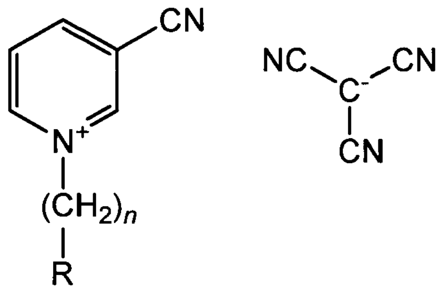

(I),

wherein *n* is an integer from 2 to 10 and R is hydrogen or -CN,
as a selective solvent in the separation of alkene-alkane mixtures.

**12.** The use of claim 11 wherein **n** is 4 and R is hydrogen.

**13.** The use of claim 11 or 12 wherein the alkene-alkane mixture is a propene-propane mixture.

Europäisches Patentamt
European Patent Office
Office européen des brevets

# EUROPEAN SEARCH REPORT

Application Number
EP 10 01 5426

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | MOKRUSHIN V ET AL: "Ionic liquids for Propene-Propane separation", CHEMICAL ENGINEERING AND TECHNOLOGY JANUARY 2010 WILEY-VCH VERLAG DEU, vol. 33, no. 1, January 2010 (2010-01), pages 63-73, XP002627535, DOI: DOI:10.1002/CEAT.200900343 * figure 1 * ----- | 1-13 | INV. C07D213/78 B01D53/14 |
| A,D | WO 2006/021390 A1 (LONZA AG [CH]; TAESCHLER CHRISTOPH [CH]; ZUR TAESCHLER CORNELIA [CH];) 2 March 2006 (2006-03-02) * example 5 * ----- | 1-13 | |
| A | WO 2010/000357 A2 (MERCK PATENT GMBH [DE]; PITNER WILLIAM-ROBERT [DE]; AUST EMIL FERDINAN) 7 January 2010 (2010-01-07) * page 25 - page 26; example G * ----- | 1-13 | |
| A | LEI Z ET AL: "Separation of 1-hexene and n-hexane with ionic liquids", FLUID PHASE EQUILIBRIA, ELSEVIER, vol. 241, no. 1-2, 15 March 2006 (2006-03-15), pages 290-299, XP024936961, ISSN: 0378-3812, DOI: DOI:10.1016/J.FLUID.2005.12.024 [retrieved on 2006-03-15] * table 3; compounds 31-33 * ----- | 1-13 | TECHNICAL FIELDS SEARCHED (IPC): C07D B01D |
| X | EP 1 854 786 A1 (BP PLC [GB]) 14 November 2007 (2007-11-14) | 10 | |
| A | * paragraph [0035]; example 15 * ----- | 1-9, 11-13 | |
| | -/-- | | |

The present search report has been drawn up for all claims

1

| Place of search | Date of completion of the search | Examiner |
|---|---|---|
| Munich | 10 March 2011 | Kollmannsberger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 01 5426

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | BUNTING J W; TOTH A; HEO C K M; MOORS R G: "Equilibration of N-(Cyanoethyl)pyridinium Cations with Substituted Pyridines and Acrylonitrile. A Change in Rate-Determining Step in an E1cb Reaction", JOURNAL OF THE AMERICAN CHEMICAL SOCIETY, vol. 112, 1990, pages 8878-8885, XP002627536, American Chemical Society ISSN: 0002-7863 * page 8879, column 2; compound 1.Br- * * table 1; compound 1a * ----- | 10 | |
| X | BAN Y ET AL: "The total synthesis of dl-epiibogamine.", TETRAHEDRON LETTERS JUN 1968 LNKD- PUBMED:5653567, vol. 30, June 1968 (1968-06), pages 3383-3386, XP002627537, ISSN: 0040-4039 * compound II * ----- | 10 | TECHNICAL FIELDS SEARCHED (IPC) |
| A | MOKRUSHIN V ET AL: "Ionic liquids for chloromethane/isobutane distillative separation: Express screening", CHEMICAL ENGINEERING AND TECHNOLOGY JUNE 2010 WILEY-VCH VERLAG DEU, vol. 33, no. 6, June 2010 (2010-06), pages 993-997, XP002627538, * synthetic procedure 2 ; page 994, column 2 * ----- | 6-9 | |

1

The present search report has been drawn up for all claims

| Place of search | Date of completion of the search | Examiner |
|---|---|---|
| Munich | 10 March 2011 | Kollmannsberger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 01 5426

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2011

| Patent document cited in search report | Publication date | Patent family member(s) | Publication date |
|---|---|---|---|
| WO 2006021390 A1 | 02-03-2006 | CN 101010291 A | 01-08-2007 |
| | | EP 1634867 A1 | 15-03-2006 |
| | | EP 1786761 A1 | 23-05-2007 |
| | | JP 2008510753 T | 10-04-2008 |
| | | KR 20070047347 A | 04-05-2007 |
| | | SG 155204 A1 | 30-09-2009 |
| WO 2010000357 A2 | 07-01-2010 | NONE | |
| EP 1854786 A1 | 14-11-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006021390 A1


### Non-patent literature cited in the description


- **N. V. PLECHKOVA ; K. R. SEDDON.** *Chem. Soc. Rev.,* 2008, vol. 37, 123-150
- **V. MOKRUSHIN et al.** *Chem. Eng. Technol.,* 2010, vol. 33, 63-73
- **SCHMIDTMANN.** *Chem. Ber.,* vol. 29, 1168-1175